# EUROPEAN PATENT SPECIFICATION

(11) **EP 1 765 291 B1**
(45) Date of publication and mention of the grant of the patent: **02.09.2009**
(21) Application number: 05748736.5
(22) Date of filing: 01.06.2005
(51) Int. Cl.: A61K 9/14, A61K 9/72, A61M 15/00

(54) **ENHANCED MEDICAL PRODUCT**
VERBESSERTES MEDIZINPRODUKT
PRODUIT MEDICAL AMELIORE

(30) Priority: 18.06.2004 SE 0401612
(43) Date of publication of application: 28.03.2007
(73) Proprietor: Mederio AG, 6052 Hergiswil NW (CH)
(72) Inventor: NIEMI, Alf, S-645 50 Strängnäs (SE); CALANDER, Sven, S-645 32 Strängnäs (SE); KAX, Lars, S-155 91 Nykvarn (SE)
(74) Representative: Stenborg, Anders Vilhelm
(86) International application number: PCT/SE2005/000822
(87) International publication number: WO 2005/123038

(56) References cited:
- EP-A1- 1 129 705
- EP-A2- 1 084 726
- US-B1- 6 263 872

## Description

### TECHNICAL FIELD

The present invention relates to a method and a medical product for enclosing a metered, dry powder medication dose together with a dose of an excipient in a common dose container intended for insertion and use in a dry powder inhaler device (DPI), resulting in an improved emitted medication dose upon inhalation. In a further aspect the invention is directed to simplifying dose forming and adapting doses for high performance inhalation.

### BACKGROUND

Within health care today administration of medicaments by inhalation for distributing dry powder medicaments directly to the airways and lungs of a user is becoming more and more popular, because inhalation offers an efficient, fast, and user friendly delivery of the specific medication substance.

Dry powder inhalers (DPIs) have become accepted in the medical service, because they deliver an effective dose in a single inhalation, they are reliable, often quite small in size and easy to operate for a user. Two types are common, multi-dose dry powder inhalers and single dose dry powder inhalers. Multi-dose devices have the advantage that a quantity of medicament powder, enough for a large number of doses, is stored inside the inhaler and a dose is metered from the store shortly before it is supposed to be inhaled. Single dose inhalers use pre-metered doses and such inhalers are loaded with a limited number of individually packaged pre-metered doses, where each dose package or container is opened shortly before inhalation of the enclosed dose is supposed to take place.

Dry powder medicaments may be in a pure formulation consisting of only active pharmaceutical ingredient (API), or the formulation may comprise other substances for different purposes, e.g. enhancing agents for increasing the bio-availability and/or bio-activity of the API. Pharmacologically inert excipients may be included for diluting a potent API, in order to act as carrier of the API or to improve the flowability of the formulation to enhance metering and filling properties of the powder.

Powders with a particle size suitable for inhalation, i.e. particles having an aerodynamic diameter, AD, in a range 0.5 - 5 µm, have a tendency of aggregating, in other words to form smaller or larger aggregates, which then have to be de-aggregated before the particles enter into the airways of the user. De-aggregation is defined as breaking up aggregated powder by introducing energy e.g. electrical, mechanical, pneumatic or aerodynamic energy. The aerodynamic diameter of a particle of any shape is defined as the diameter of a spherical particle having a density of 1 g/cm³ that has the same inertial properties in air as the particle of interest. If primary particles form aggregates, the aggregates will aerodynamically behave like one big particle in air.

The tendency to form aggregates is aggravated in the presence of water and some powders are sensitive to very small amounts of water. Under the influence of moisture the formed aggregates require very high inputs of energy to break up in order to get the primary particles separated from each other. Another problem afflicting fine medication powders is electro-static charging of particles, which leads to difficulties in handling the powder during dose forming and packaging.

Methods of dose forming of powder formulations in prior art include conventional mass, gravimetric or volumetric metering and devices and machine equipment well known to the pharmaceutical industry for filling blister packs and gelatin capsules, for example. See WO 03/66437 A1, WO 03/66436 A1, WO 03/26965 A1, WO 02/44669 A1, DE 100 46 127 A1 and WO 97/41031 for examples of prior art in volumetric and/or mass methods and devices for producing metered doses of medicaments in powder form.

Electrostatic forming methods may also be used, for example disclosed in US 6,007,630 and US 5,699,649.

Gelatin or plastic capsules and blisters made of aluminum or plastic, or laminates comprising aluminum and plastic foil are common prior art containers for metered single doses of dry powder medicaments. Typically, the user has to open the inhaler, insert at least one container into the inhaler, close it, push a button to force one or more sharp instrument(s) to penetrate a selected container, such that the dose may be accessed by streaming air when the user at leisure decides to inhale the dose. Besides a method of breaking the container open inside the inhaler and pour out the dose in a chamber first, the most common methods of opening the container are to punch one or more holes in the container itself or in a foil sealing the container or peel off the sealing foil. In the first case the powder is poured onto a surface inside the inhaler and made available for inhalation from there. In the second case the dose is aerosolized by inhalation air being forced through the container or the dose being shaken out of the container and immediately aerosolized by streaming air on the outside of the container.

However, there is still a need for improved efficacy of dry powder medicament doses being part of medical products intended for delivery by inhalation using a DPI.

### SUMMARY

The present invention relates to a method for enclosing 1) a metered, dry powder medication dose comprising at least one active pharmaceutical ingredient (API) and 2) a metered, dry powder dose of at least one excipient in 3) a common space of a common dose container. The medication formulation is a dry powder formulation adapted for inhalation and having a mass median aerodynamic diameter in a range from about 0.5 µm to about 5 µm. The excipient or excipients are biologically acceptable dry powders that are in all respects compatible with the medication powder. The invention teaches that the respective formulations are metered and filled into a common space of the dose container. The excipient or excipients have a separately metered mass calculated from a pre-defined mass-ratio relative the metered medication powder. The deposited doses in the common dose container constitute a medical product according to the invention. The method and the medical product of the invention are effective in raising the emitted medication dose output when the doses are delivered together by inhalation from a dry powder inhaler. The therapeutic efficacy of the metered medication dose is thereby improved. According to the invention, the improvement in emitted medication dose is not influenced by intentional or unintentional mixing of the doses of API and excipient after filling into the dose container, as long as the two doses are aerosolized together during inhalation. Nevertheless, arranging a somewhat random disorder of API and excipient particles by agitating the doses may be a way of raising the emitted API dose figure. Many kinds of medical dry powder formulations for inhalation benefit from the invention, such as pure API formulations, formulations comprising particles consisting of API and other ingredients and formulations of porous particles - e.g. Technospheres^{®} and microspheres In particular, the present invention is useful where the dry powder medication formulation in the dose is sticky and where particles of the medicament tend to attach themselves to surfaces with which they come in contact, such that they are difficult to set free. The present method may be advantageously applied to naturally sticky substances and medication formulations, but also to powders sensitive to ambient conditions such as elevated temperature and humidity.

### DESCRIPTION OF THE DRAWINGS

The invention will be described in the form of a preferred and illustrative embodiment and by means of the attached drawings, wherein like reference numbers indicate like or corresponding elements and wherein:
FIG. 1. illustrates in perspective (Fig. la), top (Fig. 1b) and side (Fig. 1c) views a particular embodiment of a sealed dose container filled with a dose of a medicament and a dose of an excipient;
FIG. 2 illustrates a sealed dose container filled with a dose of a medicament consisting of two deposits and a dose of an excipient consisting of three deposits.
FIG. 3 illustrates a sealed dose container after agitation filled with a dose of a medicament consisting of two deposits and a dose of an excipient consisting of three deposits where the doses have become partly mixed.
FIG. 4 illustrates in a graph results of a climate test showing the drop in fine particle dose, FPD, of Atrovent^{®} with active substance being ipratropium bromide.
FIG. 5 illustrates in a flow diagram the steps of the invention for joining a metered medication dose and an excipient dose in a common dose container.

### DESCRIPTION OF AN ILLUSTRATIVE EMBODIMENT

The present invention relates to a method for joining a metered, dry powder medication dose and a dry powder dose of a biologically acceptable excipient in a common dose container. Surprisingly, we have found that a medical product, based on the present method, improves the emitted dose, i.e. the output mass of the active ingredient of the medication powder dose, when the joined doses are inhaled from a dry powder inhaler device. The present invention improves the emitted dose by minimizing the powder retention inside the DPI. Surprisingly, we have found that the invention is advantageously applied to many types of dry powder medicament formulations intended for inhalation, particularly sticky dry powders stand to benefit. Examples of medical dry powders suitable for the present method are formulations comprising proteins, including peptides, lipids, water-soluble excipients, pure API formulations, formulations of APIs and other substances and powders of porous particles, e.g. Technospheres^{®} and microspheres. The addition of the excipient dose acts as a cleaning agent and helps to release the medication dose and entrain it into inspiration air when the doses are inhaled by use of a dry powder inhaler device. Moreover, the quantity of excipient acting as a cleaning agent in the joined doses is much less compared to what is necessary in an ordered mixture containing an API formulation and an excipient acting as diluent and carrier.

In a further aspect, it is well known in the art that many important medicaments in dry powder formulations are sensitive to high levels of humidity, such that the emitted particle dose out of an inhaler device drops drastically as the relative humidity in the air increases. This sensitivity to ambient conditions is especially noticeable among the new protein-based medicaments for inhalation under development or recently introduced into the marketplace, e.g. medicaments directed towards treatment of systemic disorders. In recent years, the pharmaceutical industry, having interests in inhalable medicaments, has directed most development resources to the formulation side of product development and the delivery systems, i.e. dose packaging and the inhaler devices, have been less in focus. Thus, the teachings herein concern how to join separate formulations of a medicament and an excipient in a medical product, which provides improvements in drug delivery performance by inhalation and thereby improved therapeutic efficacy.

A successful formulation of an API for inhalation needs to be inter alia chemically and biologically stable under storage and in-use conditions, it needs to have a high bio-availability and bio-activity, a suitability for a filling process and a narrow particle size distribution. There are a number of well-known techniques for obtaining a suitable primary particle size distribution that will ensure correct lung deposition for a high percentage of the dose mass. Such techniques include jet-milling, spray-drying and super-critical crystallization. There are also a number of well-known techniques for modifying the forces between the particles and thereby obtaining a powder with suitable adhesive forces. Such methods include modification of the shape and surface properties of the particles, e.g. porous particles and controlled forming of powder pellets, as well as addition of an inert carrier with a larger average particle size (so called ordered mixture). A simpler method of producing a finely divided powder is milling, which produces crystalline particles, while spray-drying etc produces generally more amorphous particles. Novel drugs, both for local and systemic delivery, often include biological macromolecules, which do add completely new demands on the formulation and the production process. Examples of problems, which need to be addressed when developing a formulation for inhalation comprising an API and optionally other substances, are:
- API stability
- Absorption of the API in the lung
- Solubility of the API
- Particle size distribution
- Dilution of API potency
- Elimination of unpleasant taste
- Powder flowability

When a working formulation has been developed and regulatorily approved together with a chosen packaging and dose delivery system, the threshold of improving the formulation chemically or biologically is very high, because the whole regulatory process must be repeated. Besides the time and cost involved in developing a new formulation, most time and money will be spent on regulatory work. From this aspect, the present invention may provide a fast road to higher medical efficacy by making a switch to a different technical platform possible. Technically, it is very straightforward to implement the present invention and to switch the packaging and dose delivery systems. A new dose container may be developed or an existing one may be chosen capable of accepting a dose of the original formulation and a dose of a selected excipient, thereby constituting a medical product according to the invention. Examples of suitable DPIs, which may be used with the present invention are described in our publications US 6,622,723 and US 6,422,236. Regulatorily, combining a well-known, proven formulation with a biologically acceptable excipient does not require extensive development and clinical studies to aquire an approval. The regulatory process is normally in such cases uncomplicated and quick in comparison.

Preferably, the de-aggregating system should be as insensitive as possible to variations in the inhalation effort produced by the user, such that the delivered aerodynamic particle size distribution in the inhaled air is largely independent of the inhalation effort over a certain minimum level. A very high degree of de-aggregation presumes the following necessary steps:
- a suitable formulation of the powder (particle size distribution, particle shape, adhesive forces, density, etc)
- a suitably formed dose of the powder adapted to the capabilities of a selected inhaler device
- an inhaler device providing shear forces of sufficient strength in the dose to release and de-aggregate the powder (e.g. turbulence)
   A method and a device for de-aggregating a powder is disclosed in our US patent No. 6,513,663 B1.

### Dose forming

An advantage of the present invention is that prior art methods of dose forming of medication and excipient powder formulations for inhalation are easily applied, such as conventional mass, gravimetric or volumetric dose metering. Filling devices and machine equipment well known to the pharmaceutical industry for filling blister packs and gelatin capsules, for example, may be used. Electrostatic forming methods may also be used, for example as disclosed in our publication WO 02/11803 (US 6,696,090) disclosing a method and a process of preparing a so called electro-powder, suitable for forming doses by an electro-dynamic method, further described in our publication US 6, 868,853, which both are incorporated hereby in this document in their entirety by reference. These disclosures stress the importance of controlling the electrical properties of a medication powder and points to the problem of moisture in the powder and the need of low relative humidity in the atmosphere during dose forming.

Suitable dose sizes for inhalation are typically in a total mass range from 1 mg to 20 mg. Smaller doses than 1 mg are difficult to meter and fill consistently and doses having a mass exceeding 20 mg may be difficult to release and de-aggregate completely in the DPI. Many of the new protein-based active substances require a metered mass of the API in the order of 1 - 5 mg to give the desired therapeutic effect when inhaled. If the medicament comprising the API is a candidate for being included in a mixture, further comprising an excipient of bigger particles, typically of average size between 20 and 200 µm, acting as carriers of the medicament, one must keep in mind that a stable, homogenous, ordered mixture in bulk quantity that does not begin to segregate when used in a repetitive, filling operation, cannot hold more than 4 - 5 % by weight (w/w) of the medicament. Segregation means that small drug particles separate from the big excipient ones, leading to different concentrations of the API in different parts of the bulk powder store. Given that the medicament mass is in the range 1-5 mg, i.e. pure API having a therapeutic effect, a metered dose of an ordered mixture will be in a range from 20 to 125 mg. A dose mass in this range is not suitable for inhalation. APIs for systemic absorption by pulmonary delivery must have particles in a range 1-3 µm, which makes it difficult to make a homogenous, ordered mixture which does not segregate when later used in a filling process. Anyone will realize that if the concentration of API varies in the bulk powder mixture and if segregation occurs during handling and in the filling process, it will be impossible to know how much API drug that is filled each time. A particular aspect of the present invention presents a solution to this problem by using far less excipient, not in an ordered mixture with the medicament as in prior art, but separately dosed into the same dose container as the medicament dose.

The present invention uses ratios API/excipient in a range 1/20- 20/1. In fact, the present invention simplifies the dose filling in many cases, because the complexity of making a stable mixture of the API formulation and a suitable excipient is eliminated. A first dose of the formulation containing the API is metered and filled into a dose container and a second dose of at least one excipient is also filled into the same dose container. The order of filling the two doses is irrelevant to the invention. A best mode of filling depends e.g. on the formulations, the selected mass ratio, the dose container and the DPI, which will receive the medical product containing the doses to be inhaled. If the medication dose and the excipient dose are deposited separately in a common container, which is then sealed, agitating the container indefinitely will not result in a homogenous mixture, as samples will show. Nevertheless, in a particular embodiment, agitating the dose container after filling with API and excipient doses will create a non-uniform mixture, which will boost the emitted dose of the API. However, agitating the dose container may be preferred, but is not necessary, in order to implement the invention, provided the doses are arranged to be inhaled simultaneously together. An advantage of the invention is that the road to regulatory approval may be considerably shorter compared to taking a new formulation through the necessary, regulatory steps. A further advantage of the disclosure is that metering and filling of the medicament dose may become simpler compared to filling an ordered mixture. Both the cleaning excipient and the medicament are often more easily separately metered.

Generally, dry powder medicament doses need to be protected by an enclosure not only during storage, but also when inserted in an inhaler, e.g. a single dose DPI, where the dose and its enclosure are kept in a ready state before delivery in an inhalation at a point in time decided by the user. New types of dry powder medicaments, not least for systemic treatment, have a rather short expiry date and they are generally quite sensitive to ambient conditions, especially moisture during storage and in use. Hence, the demands put on dose protection and inhaler devices in handling sensitive doses are therefore much higher than for prior art devices as used e.g. for administering traditional medicaments against respiratory disorders.

In, the development of new and improved types of single dose dry powder inhalers, see our U.S. Patent No's 6,622,723, 6,422,236, 6,868,853, 6,571,793 and 6,840,239, we have also developed dose filling methods, see our U.S. Patent No. 6,592,930 and WO 04/110539, all of which are incorporated in this document in their entirety by reference. In the development work, particular attention has been devoted to sticky substances per se, i.e. such dry powders which are inclined to leave a high percentage of the active substance, the active pharmaceutical ingredient(s), retained on the inner surfaces of the dose container or aerosolization chamber and on the internal walls of the air channels inside the inhaler device, through which the airflow passes carrying the released dose intro the airways of the user. Besides stickiness in the active substance, i.e. the API, stickiness may be experienced when using easily water-soluble excipients in the formulation or when the powder formulation consists of porous particles where the surface area of a particle is very large relative the particle's mass. Stickiness in a powder may also be a result of particles disposed to form large particles, which sometimes also are difficult to de-aggregate. Not surprisingly, if the humidity of the surrounding air is high, we've found that medical powders in general are more inclined to stick to any surface with which they come in contact. What degree of air humidity is deemed to be high depends on the sensitivity to humidity of the powder. The retention effect for a sticky powder depends, inter alia, on the structure of surfaces in contact with the powder, the surface areas, the materials concerned, the design of the inhaler device and the aerosolization and de-aggregation forces provided by the DPI, to name a few important factors. Time is another factor e.g. when stickiness is due to high moisture. Different powders adsorb more or less humidity and at different rates. However, many medical powders are affected within milliseconds of being exposed to humidity in the ambient air. Other powders adsorb water more slowly. In any case, it is not satisfactory having an inhaler designed such that the user may open a dose container first, allowing the ambient atmosphere access to the dose therein for an undefined time period of seconds or even minutes before an inhalation commences.

Surprisingly, we have found that a low figure of emitted dose from a DPI may be drastically improved by introducing a suitable, biologically inert excipient in dry powder form into the dose container or chamber at the dose metering and filling stage, thereby joining a dose of the medication in question to a dose of the excipient. Naturally, the excipient or excipients must be compatible in all respects with the medication powder. For best accuracy, the invention teaches that the respective formulations are to be separately metered and filled into the same dose container, where they intentionally or unintentionally may or may not be mixed after filling. The doses must, however, be so arranged in the dose container that they will be aerosolized simultaneously together. The improvement in emitted dose as a percentage of the metered dose is very significant. Particularly sticky powders may benefit from the present invention, because the relative improvement in emitted dose may be more significant than for more easily aerosolized powders. Powders may be naturally sticky or conditionally sticky or both, e.g. if affected by humidity. The excipient may be separately filled into the dose container before or after filling of the medicament dose. Surprisingly, we have found that even a small amount of a selected, suitable excipient powder, which is used to coat the inner surfaces of the container prior to dose filling, may be sufficient to raise the emitted dose figure of the active pharmaceutical ingredient, API. From a filling point of view, however, a coating of the container internal surfaces presents many problems, such as the risk of unintentional spreading of excipient particles onto sealing surfaces of the container, thereby risking the sealing quality. It is preferred to fill the container with doses of the medicament and the excipient, where each dose consists of at least one metered deposit of the formulation in question. When the dose container later is opened and the doses aerosolized during an inhalation, the particles of the excipient dose act as cleaning agents for the container and the internal parts of the inhaler, whereby a high share of the medication powder particles that stick to the interior surfaces are forcibly released and entrained in the streaming inhalation air. The clensing effect is very obvious whether or not the medication dose has been mixed with the excipient dose prior to an inhalation, provided the doses are released simultaneously together. However, a best mode of filling the API and the excipient in a selected dose container and what mass ratio to use between medicament and excipient must be decided during development of the particular medical product. Different formulations do not necessarily benefit from the same filling method and the optimum quantity of excipient dose to be deposited into the dose container together with the medication dose depends on the formulation of the API, among other things. In a particular embodiment a dose comprising at least one powder deposit of the medicament is deposited in the dose container and deposits of the excipient are deposited on diametrically opposed sides of the medication deposits. The deposits of the excipient are preferably of approximately the same mass and the deposits added together constitute the excipient dose. Typically, the dose mass of the excipient is roughly the same as the mass of the medication dose. The deposition sequence and pattern of the deposits making up the respective doses in the dose container depend on how the DPI aerosolizes the powder in the dose container. The deposited excipient dose must be properly aerosolized like the medication dose, or else the cleaning effect of the excipient may be less efficient.

In another embodiment of the invention, a dose comprising at least one API and a dose comprising at least one biologically acceptable excipient are separately metered, whereupon the metered doses are filled together into a dose container, optionally first being at least partly mixed prior to filling.

In a further particular embodiment, a dose of excipient is filled into the container in a first step, but not spread out inside. Then, in a second step the medicament is filled into the container, optionally on top of the excipient. Optionally, a further amount of excipient is deposited on top of the dose. A further option is to agitate by e.g. shaking or vibrating the dose container after filling and optionally after sealing of the container, whereby the excipient(s) become roughly mixed with the medicament powder. The non-uniform mixture is characterized in that it does not constitute an ordered mixture. We have surprisingly found that even if the excipient, acting as a cleaning agent, does not isolate the dose from contacting the internal surfaces of the container, still the excipient manages to clean out the dose from the container. When the powder in the container or aerosolization chamber is attacked by a sufficiently turbulent air-stream, the medicament powder dose is released, de-aggregated and entrained into the air-stream that flows through the inhaler air channels and further into the airways of a user. Besides acting as carriers of small particles, it is assumed that mainly the coarse excipient particles act similarly to a sandblasting device, i.e. to physically set medicament particles free by sheer impaction power. Which combination of excipients, particle sizes and methods of depositing amounts of excipients depends on the excipient(s) and the medicament. What degree of agitation to use, if at all, must also be investigated in each individual application.

The excipient may comprise fine particles ≤ 10 µm, particles ≥ 10 µm or the excipient may comprise fine particles ≤10 µm and coarse particles ≥10 µm. The excipient particles having an aerodynamic diameter (AD) of 10 µm or more are deposited by impaction in the mouth, throat and upper airways upon inhalation, because the mass of these excipient particles is generally too big to follow the inspiration air into the lung. Therefore, excipients are selected inter alia with a view to be harmless when deposited in the areas concerned. However, a formulation of excipient(s) may comprise more than one excipient. For instance it is often advantageous to include an excipient consisting of small particles ≤ 10 µm in a mixture with an excipient consisting of big particles ≥ 10 µm, more typically ≥25 µm. This mixture flows easily and a metered dose of the mixture holds together when lightly compacted and makes the filling process simple. The mass ratio between small particles and big ones is in a range 0.01 - 0.1 and typically 0.02 - 0.05 for best operation. The excipients used may or may not be of the same substance.

Suitable excipients for inclusion in a dose container are to be found among the groups of monosaccarides, disaccarides, polylactides, oligo- and polysaccarides, polyalcohols, polymers, salts or mixtures from these groups, e.g. glucose, arabinose, lactose, lactose monohydrate, lactose anhydrous [i.e., no crystalline water present in lactose molecule), saccharose, maltose, dextrane, sorbitol, mannitol, xylitol, sodium chloride, calcium carbonate. A particular excipient is lactose. Lactose in a dry powder form, so called Respitose^{®} from DMV International having 95 % of particles larger than 32 µm, has been successfully used as a cleaning excipient in many inhalation experiments of ours.

The moisture properties of any proposed excipient must be checked before it is chosen to be used as a cleaning agent. If an excipient gives off water, after dose forming, it may negatively affect the API in the medicament dose, such that the fine particle dose, FPD, deteriorates rapidly after sealing of the dose container. Therefore, excipients to be in contact with or to be mixed with the medicament are to be selected among acceptable excipients, which have good moisture properties in the sense that the excipient will not adversely affect the FPD of the API(s) for the shelf life of the product, regardless of normal changes in ambient conditions during transportation and storage. Suitable "dry" excipients are to be found in the above-mentioned groups. In a particular embodiment of the present invention, lactose is selected as the preferred dry excipient and preferably lactose monohydrate. A reason for selecting lactose as excipient, is its inherent property of having a low and constant water sorption isotherm. Excipients having a similar or lower sorption isotherm can also be considered for use, provided other required qualities are met.

Ambient conditions during dose metering, filling and container sealing should be closely controlled. The ambient temperature is preferably limited to 25°C maximum and relative humidity preferably limited to 15 % Rh maximum, but the actual permissible relative humidity depends on the specific formulation and some cases may require much less than 15 % Rh, even less than 5 % Rh. The powder formulation is also to be kept as dry as possible during the dose forming process. Further, it is very important to control the electric properties of the powders and to apply electric charging and discharging as needed, regardless of which method of dose forming is to be used. Fine powders pick up static electric charges extremely easily, which can be advantageously used in dose forming, if the charging and discharging is under proper control. Keeping the ambient relative humidity low ensures that only a very small, acceptable amount of water is enclosed in the dose container together with the dose and not enough to present a threat to the stability of a moisture sensitive substance and the FPD of the dose.

The disclosed method counteracts as far as possible any adverse influence that e.g. humidity in the air may have on the fine particles in the dose. Minimizing the dose exposure to the atmosphere may preferably be done by implementing a breath actuation mechanism coupled to opening of the dose container in the inhaler. But the present invention may be advantageously used to boost performance from a dry powder inhaler device. Examples of problems in prior art dry powder inhaler devices, having negative effects on the emitted dose are:
- Inhaler design provides too low airflow turbulence close to dose during inhalation;
- Selected dose container is bigger than necessary and provides too much internal surface area for powder to stick to;
- Inhaler and dose container present high sticking effects between dose particles and internal surfaces of dose container and inhaler;
- User interface of the inhaler gives ambient humid air access to the dose for a long time before an inhalation actually takes place;
Such failings in prior art DPI devices may be rendered less detrimental and the emitted dose is improved by the adoption of the present invention. Most preferably, however, the present invention is applied in an inhaler incorporating an Air-razor device for a gradual dose release in a prolonged dose delivery period, as described in our U.S. Patent No. 6,840,239.

### Example 1: Mixtures of API and Excipient

In the course of developing methods and products according to the present invention different APIs were mixed with excipients in different ratios. The objectives were to find inter alia suitable formulations and methods of filling doses, but also to optimize inhaler techniques and interaction between inhaler and dose container. Table 1 below discloses some examples of volumetrically dosed mixtures API/excipient and the resulting emitted dose when delivered by a proprietary DPI.

**Table 1**

| **API** | **Excipient** | **Ratio API/Excipient** | **Emitted Dose in % of recovered dose** | **Retention in % of recovered dose** |
|---|---|---|---|---|
| Micronized insulin | Mannitole | 50/50 | 83 | 17 |
| Micronized insulin | Respitose^{®} | 20/80 | 93 | 7 |
| Fluticasone | Respitose^{®} + 10 % micronized lactose | 1/44 | 88 | 12 |

### Conclusions

As shown in Table 1, mixtures with far more micronized API (insulin) than 5 % were used in these tests. The objective was to find suitable excipients and to see how different mixing ratios affected the emitted dose. The mixtures were produced under laboratory conditions in small quantities and remained quasi-stable under the run of tests. The emitted dose as percentage of total recovered dose was measured and the results were used in the development of the current invention.

### Example 2: Ipratropium climate stability tests

This test was made in order to find out how sensitive ipratropium bromide is to moisture. Commercially available Atrovent ^{®} capsules containing ipratropium bromide and excipient were bought in from our local pharmacy and introduced into the laboratory together with the HandiHaler^{®} dry powder inhaler device. The powder was withdrawn from the originator's capsules and transferred to the capsules again after climate storage. The aerodynamic fine particle fraction (FPF) in the emitted dose from the HandiHaler^{®} was measured using Andersen impactors according to European Pharmacopoeia (EP) and US Pharmacopoeia (USP). All analytical work then was performed according to standardized methods using a state of the art High Performance Liquid Chromatograph (HPLC) system.

### Test S1

Aerodynamic fine particle fraction of metered and delivered dose out of Handihaler^{®} using Atrovent^{®} formulation powder was analyzed. Transfer of powder from and back into originator capsules was performed in relative humidity below 10 %. The test was performed with 4 kPa pressure drop over the HandiHaler^{®} at room temperature and laboratory ambient conditions.

### Test S2

An in-use stability test was carried out of the aerodynamic fine particle fraction of metered and delivered dose out of Handihaler^{®}. From the blister holding the Atrovent^{®} capsules the powder was transferred to a medium moisture barrier container and sealed. The containers were put for 1 month in 25°C and 60 % Rh. The container holding the powder was then put in an exicator for 2 h before tests were performed. The inhaler test was performed with 4 kPa pressure drop over the HandiHaler^{®} at room temperature and laboratory ambient conditions.

### Test S3

The same test as in S2 was carried out except that the containers were put for 1 month in 40°C and 75 % Rh.

### Conclusions

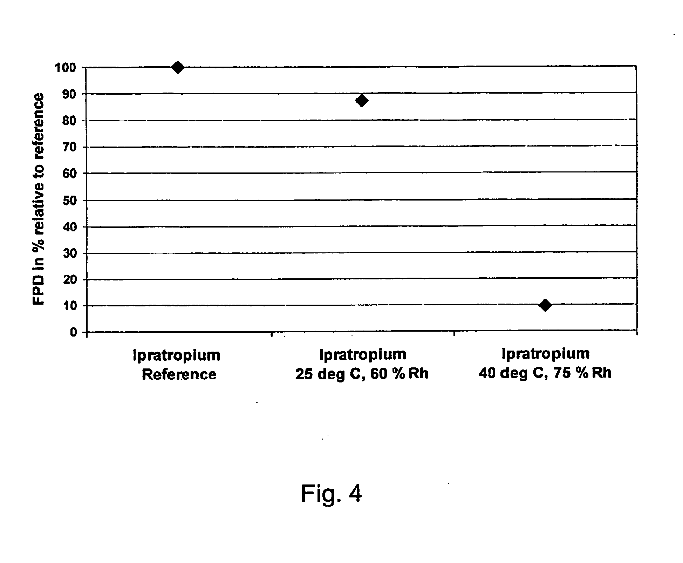

It is obvious from the graph in Figure 4 showing the drop in fine particle dose, FPD, that ipratropium bromide is a very moisture sensitive substance.

### Example 3

In order to illustrate the positive effect on emitted dose, i.e. the mass of the medicament powder entrained in inspiration air leaving a typical DPI, the following tests were carried out in our laboratory.

A pure, micronized, recombinant, human insulin in dry powder form was selected as the medicament test substance. Lactose in a dry powder form, so called Respitose^{®} from DMV International having 95 % of particles larger than 32 µm, was selected as a cleaning excipient.

Four dose containers, aluminum blisters constituting so called pods, were filled in a dry climate with nominally 2.5 mg insulin. The filled containers were designated 'A'.

Four further containers, identical to the first four, were filled in the same, dry climate with nominally 2.5 mg insulin and 2.5 mg Respitose^{®}, in separate filling steps, making a total dose of 5 mg and a mass ratio of 50/50 between insulin and Respitose^{®}. The filled containers were designated 'B'.

The containers were adapted for insertion into a proprietary, single dose DPI, called E-flex.

Two containers of type 'A' and two containers of type 'B' were put for an hour before testing in a climate cabinet set at room temperature and approximately 90 % relative humidity and the remaining containers were stored in the laboratory under normal ambient conditions.

The emitted doses were measured using a total of four DPI test devices, one per type of filling ('A' and 'B') and climate. Emitted dose was measured using a HPLC analyzer. Retention in the containers and in the suction tube and mouthpiece of the inhalers were also measured using the HPLC analyzer. Results are presented in Table 2 below.

**Table 2**

| **Type of filling** | **Emitted Dose in % of metered dose** | **Retention in % of metered dose** | **Emitted dose, µg** | **Retention, µg** |
|---|---|---|---|---|
| Ambient'A' | 87 | 13 | 2166 | 320 |
| Ambient B' | 93 | 7 | 2296 | 185 |
| Humid 'A' | 76 | 24 | 1982 | 610 |
| Humid 'B' | 79 | 21 | 1992 | 543 |

### Conclusions from climate testing

The tests show conclusively that the cleaning excipient Respitose^{®} boosts the emitted dose (ED) leaving the inhaler. This is especially noticable under ambient conditions where the ED increases from 87 to 93 % of the metered dose and retention is reduced by almost 50 % from 13 to 7 %. In the humid case a 3 % improvement is seen , retention is reduced by approximately 10 - 15 %, from 24 to 21 %. It is also interesting to note that the efficacy of the test DPI system is very high even under extremely humid conditions.

The disclosed method must be adapted to the particular type of dose container, which has been selected for insertion into a particular, adapted dry powder inhaler. As already pointed out, different types of dose containers are advantageously used in the present invention. Examples of containers are aluminum or plastic single dose blisters of varying size and design and also capsules of gelatin, cellulose or plastics.

Prior art blister packages for dry powder medicaments, intended for inhaler use, often have a fairly thin polymeric seal, which can be easily ripped or punched open before the dose is supposed to be inhaled. Another common seal is a peelable foil such that the blister is peeled open prior to inhalation of the enclosed dose. Yet another type of prior art dose container is the capsule. Capsules are often made of gelatin, but polymers and cellulose and other materials are also used. A common problem for prior art blisters and capsules used for dry powder doses for inhalation is that the primary package does not protect sensitive substances from moisture well enough during storage and in use. Minimizing the time the primary package is exposed to the atmosphere and minimizing the time during which the dose is subjected to the ambient atmosphere after opening of the container are therefore important aspects of inhaler and dose container design.

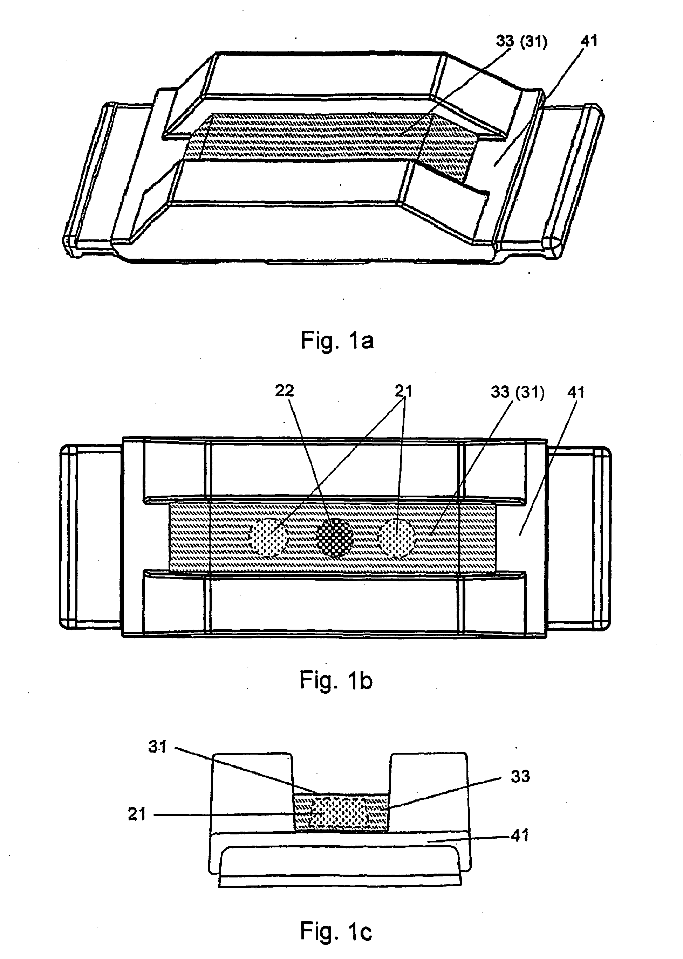

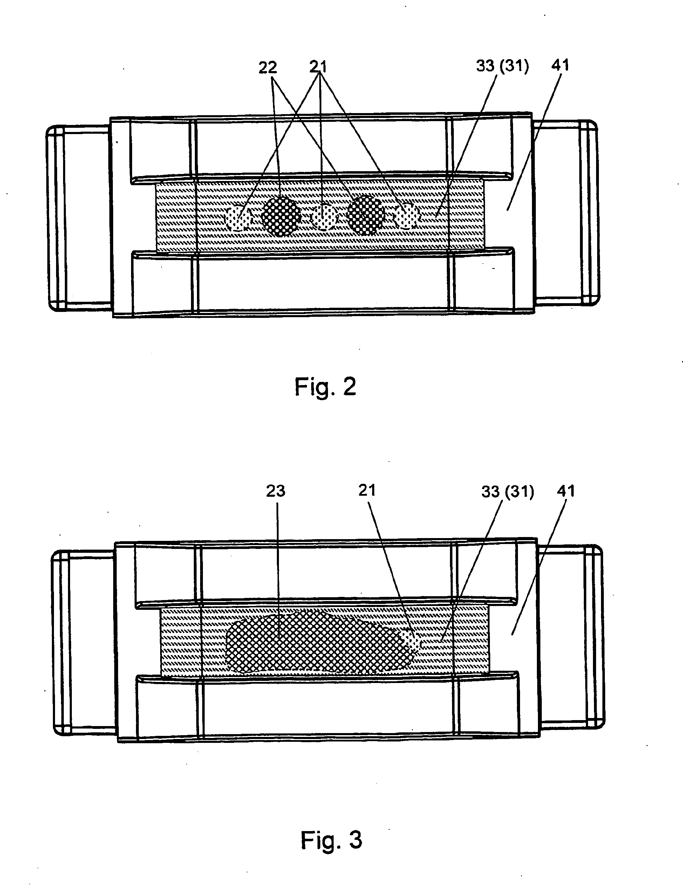

Using a new type of blister pack, a so-called pod (patent pending), as a particular embodiment of a sealed dose container, is to be preferred in an application where the present invention is to be put to use. A pod container may be made as a high barrier seal container offering a very high level of moisture protection and which is in itself dry, i.e. it does not contain water. See Figure 1 illustrating a pod carrying a sealed container in a perspective drawing. Figure la shows a sealed container 33 (seal 31) put into a protective casing 41 adapted for insertion into a dry powder inhaler. Figure 1b shows a top view of the carrier/container and indicates a dose of a dry powder medicament 22 and a dose of a dry powder excipient consisting of two depositions 21 inside the container 33 under a seal 31. Figure 1c illustrates a side view of the carrier/container in Figure 1b. Figure 2 illustrates a similar container to Figure 1, but the medicament dose consists of two deposits 22 and the excipient dose consists of three deposits 21.

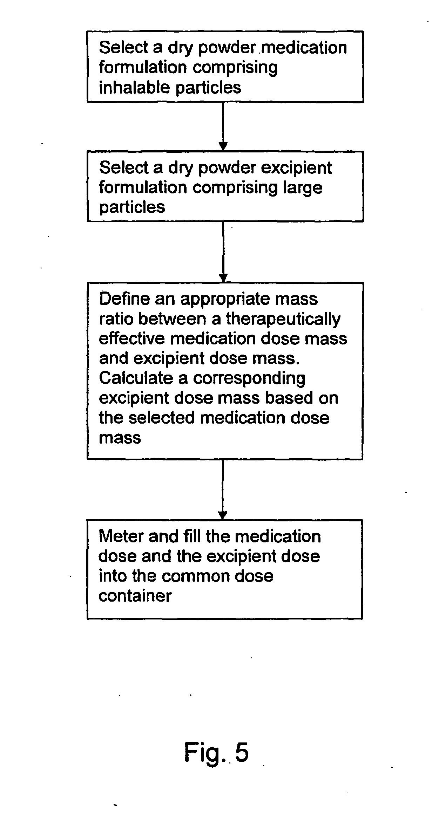

Figure 3 illustrates the dose container in Figure 2 after agitation of the container, whereby the deposits 21 and 22 have become partly mixed in a load 23. Figure 5 illustrates in a flow diagram the steps of the present invention for joining a metered medication dose and an excipient dose in a common dose container.

The invention teaches that the addition of an excipient dose to a medication dose at the inhalation stage improves the release of the API of the medication powder dose, such that the emitted API dose increases and the retention in the dose container and in the down stream airflow channels decreases. It is not necessary to arrange a mixing of the doses, as long as the excipient dose generally is aerosolized simultaneously together with the medication dose. The excipient dose mass is not critical to achieve an improvement in the quantity of the emitted API dose. The big excipient particles will impact and stick in the mouth and throat and become swallowed and will have no detrimental effect on the efficacy of the emitted dose.

It will be understood by those skilled in the art that various modifications and changes may be made to the present invention without departing from the scope thereof, which is defined by the appended claims.

## Claims

1. A method of joining a metered, dry powder medication dose, comprising at least one active pharmaceutical ingredient, together with a dry powder excipient dose, comprising at least one biologically acceptable excipient, in a common dose container, said doses intended for delivery by use of a dry powder inhaler device, **characterized by** the steps of
selecting a formulation of the dry powder medication dose consisting of inhalable powder particles having a mass median aerodynamic diameter within a range from about 0.5 µm to about 5 µm;
selecting a formulation of the at least one excipient comprising large particles to at least 90 % by mass;
defining an appropriate mass ratio between a selected, therapeutically effective medication dose mass to be filled and the dose mass of the excipient, whereupon the corresponding excipient dose mass is calculated,
separately metering the selected, therapeutically effective medication dose and the calculated dose of the excipient, and
filling the metered selected, therapeutically effective medication dose and the metered calculated dose of the excipient, optionally by making one or more depositions per dose, into the common dose container.

2. The method according to claim 1, **characterized by** the further step of
mixing the medication and the excipient doses after an individual metering operation but prior to depositing the metered doses into the dose container, whereby the doses are already at least partly mixed when deposited in said container.

3. The method according to claim 1, **characterized in that** said filling step comprises the step of
separately filling the metered selected, therapeutically effective medication dose and the metered calculated dose of the excipient, optionally by making one or more depositions per dose, into the common dose container.

4. The method according to any of the claims 1 to 3, **characterized by** the further step of
agitating the dose container holding the metered doses using electrical or mechanical energy such that the doses inside the container become at least partly mixed.

5. The method according to claim 1, **characterized by** the further step of
selecting the at least one excipient from a group consisting of monosaccarides, disaccarides, polylactides, oligo- and polysaccarides, polyalcohols, polymers, salts or mixtures thereof.

6. The method according to claim 1 or 5, **characterized by** the further steps of
selecting a formulation of the at least one biologically acceptable excipient comprising large particles bigger than 20 µm in size to at least 90 % by mass.

7. The method according to claim 1, **characterized by** the further step of
defining the mass ratio between the medication dose and the excipient dose to be in a range 1:20 - 20:1.

8. The method according to claim 1, **characterized by** the further step of
sealing the common dose container moisture-tight by using a high barrier seal.

9. A medical product comprising a dose container enclosing a dry powder medication dose, comprising at least one active pharmaceutical ingredient, and further enclosing a dry powder excipient dose, comprising at least one biologically acceptable excipient, said doses suitable for inhalation from the dose container by use of a dry powder inhaler device, **characterized in that**
the medication dose has a separately metered, therapeutically effective mass, said dose consisting of powder particles of a mass median aerodynamic diameter in a range from about 0.5 µm to about 5 µm;
the excipient dose has a separately metered mass calculated from a pre-defined mass-ratio relative the metered medication dose; and
said medication and excipient doses, optionally split up in more deposits than one per dose in the common dose container, are arranged for a simultaneous release together upon an inhalation using the dry powder inhaler device.

10. The medical product according to claim 9, **characterized in that**
the dose container holding the metered doses is agitated using electrical or mechanical energy such that the doses inside the container become at least partly mixed.

11. The medical product according to claim 9, **characterized in that**
a mass ratio between the metered medication dose and the excipient dose is selected to be in a range 1:20 - 20:1.

12. The medical product according to claim 9, **characterized in that**
the at least one excipient is selected from a group consisting of monosaccarides, disaccarides, polylactides, oligo- and polysaccarides, polyalcohols, polymers, salts or mixtures thereof.

13. The medical product according to claim 9, **characterized in that**
the at least one excipient consists to at least 90 % by mass of particles 20 µm in size or bigger and optionally particles of sizes ranging between 0.5 and 10 µm.

14. A use of the medical product according to claim 8 9, **characterized in that**
an enhanced and consistent delivery of the active pharmaceutical ingredient of the medication dose is achieved and retention of the active pharmaceutical ingredient is minimized in any selected dry powder inhaler device where the medical product is applied.

## Patentansprüche

1. Verfahren zum Zusammenführen einer abgemessenen Trockenpulver-Medikationsdosis, umfassend zumindest einen aktiven pharmazeutischen Inhaltsstoff, mit einer Trockenpulver-Trägerstoffdosis, umfassend zumindest einen biologisch akzeptablen Trägerstoff, in einem gemeinsamen Dosisbehälter, wobei die Dosen zur Verabreichung mittels Verwendung einer Trockenpulver-Inhalatorvorrichtung vorgesehen sind, **gekennzeichnet durch** die Schritte:
Auswählen einer Rezeptur der Trockenpulver-Medikationsdosis, bestehend aus inhalierbaren Pulverteilchen mit einem Median des aerodynamischen Massendurchmessers innerhalb eines Bereichs von ca. 0,5 µm bis ca. 5 µm;
Auswählen einer Rezeptur des zumindest einen Trägerstoffs, umfassend große Teilchen bis zumindest 90 Masse%;
Definieren eines geeigneten Masseverhältnisses zwischen einer ausgewählten, therapeutisch wirksamen Medikationsdosismasse, die eingefüllt werden soll, und der Dosismasse des Trägerstoffs, woraufhin die entsprechende Trägerstoffdosismasse berechnet wird, getrenntes Abmessen der ausgewählten, therapeutisch wirksamen Medikationsdosis und der berechneten Dosis des Trägerstoffs, und
Einfüllen der abgemessenen, ausgewählten, therapeutisch wirksamen Medikationsdosis und der abgemessenen berechneten Dosis des Trägerstoffs, optional **durch** Herstellen einer oder mehrerer Depots pro Dosis, in den gemeinsamen Dosisbehälter.

2. Verfahren nach Anspruch 1, **gekennzeichnet durch** den weiteren Schritt:
Mischen der Medikationsdosis und der Trägerstoffdosis nach einem individuellen Abmessvorgang, aber vor dem Deponieren der abgemessenen Dosen in den Dosisbehälter, wobei die Dosen bereits zumindest teilweise gemischt sind, wenn sie in dem Behälter deponiert werden.

3. Verfahren nach Anspruch 1, **dadurch gekennzeichnet, dass** der Einfüllschritt den Schritt umfasst:
getrenntes Einfüllen der abgemessenen, ausgewählten, therapeutisch wirksamen Medikationsdosis und der abgemessenen berechneten Dosis des Trägerstoffs, optional durch Herstellen einer oder mehrerer Depots pro Dosis, in den gemeinsamen Dosisbehälter.

4. Verfahren nach einem der Ansprüche 1 bis 3, **gekennzeichnet durch** den weiteren Schritt:
Schütteln des Dosisbehälters, der die abgemessenen Dosen enthält, unter Verwendung elektrischer oder mechanischer Energie, so dass die Dosen innerhalb des Behälters zumindest teilweise gemischt werden.

5. Verfahren nach Anspruch 1, **gekennzeichnet durch** den weiteren Schritt:
Auswählen des zumindest einen Trägerstoffs aus einer Gruppe bestehend aus Monosacchariden, Disacchariden, Polyactiden, Oligo- und Polysacchariden, Polyalkoholen, Polymeren, Salzen oder Mischungen derselben.

6. Verfahren nach einem der Ansprüche 1 bis 5, **gekennzeichnet durch** den weiteren Schritt:
Auswählen einer Rezeptur des zumindest einen biologisch akzeptablen Trägerstoffs, umfassend große Teilchen größer als 20 µm in der Größe bis zumindest 90 Masse%.

7. Verfahren nach Anspruch 1, **gekennzeichnet durch** den weiteren Schritt:
Definieren des Masseverhältnisses zwischen der Medikationsdosis und der Trägerstoffdosis auf einen Bereich zwischen 1:20 bis 20:1.

8. Verfahren nach Anspruch 1, **gekennzeichnet durch** den weiteren Schritt:
feuchtigkeitsdichtes Versiegeln des gemeinsamen Dosisbehälters **durch** Verwendung einer starken Sperrabdichtung.

9. Medizinisches Produkt, umfassend einen Dosisbehälter mit einer Trockenpulver-Medikationsdosis, umfassend zumindest einen aktiven pharmazeutischen Inhaltsstoff, und ferner mit einer Trockenpulver-Trägerstoffdosis, umfassend zumindest einen biologisch akzeptablen Trägerstoff, wobei die Dosen zur Inhalation vom Dosisbehälter mittels einer Trockenpulver-Inhalatorvorrichtung vorgesehen sind, **dadurch gekennzeichnet, dass**
die Medikationsdosis eine getrennt abgemessene, therapeutisch wirksame Masse aufweist, wobei die Dosis aus Pulverteilchen mit einem Median des aerodynamischen Massendurchmessers in einem Bereich von ca. 0,5 µm bis ca. 5 µm besteht;
die Trägerstoffdosis eine getrennt abgemessene Masse aufweist, die aus einem vorab definierten Massenverhältnis in Bezug auf die abgemessene Medikationsdosis berechnet wird; und
die Medikationsdosis und die Trägerstoffdosis, optional in mehr Depots als einem pro Dosis in dem gemeinsamen Dosisbehälter aufgeteilt, für eine gleichzeitige gemeinsame Abgabe bei einer Inhalation unter Verwendung der Trockenpulver-Inhalatorvorrichtung angeordnet sind.

10. Medizinisches Produkt nach Anspruch 9, **dadurch gekennzeichnet, dass** der Dosisbehälter, der die abgemessenen Dosen enthält, unter Verwendung elektrischer oder mechanischer Energie geschüttelt wird, so dass die Dosen innerhalb des Behälters zumindest teilweise gemischt werden.

11. Medizinisches Produkt nach Anspruch 9, **dadurch gekennzeichnet, dass** ein Masseverhältnis zwischen der abgemessenen Medikationsdosis und der Trägerstoffdosis so ausgewählt ist, dass es in einem Bereich zwischen 1:20 bis 20:1 liegt.

12. Medizinisches Produkt nach Anspruch 9, **dadurch gekennzeichnet, dass** der zumindest eine Trägerstoff aus einer Gruppe bestehend aus Monosacchariden, Disacchariden, Polyactiden, Oligo- und Polysacchariden, Polyalkoholen, Polymeren, Salzen oder Mischungen derselben ausgewählt wird.

13. Medizinisches Produkt nach Anspruch 9, **dadurch gekennzeichnet, dass** der zumindest eine Trägerstoff bis zumindest 90 Masse% aus Teilchen mit einer Größe von 20 µm oder größer und optional aus Teilchen in Größen im Bereich zwischen 0,5 und 10 µm besteht.

14. Verwendung des medizinischen Produkts nach Anspruch 9, **dadurch gekennzeichnet, dass** eine verbesserte und gleichmäßige Verabreichung des aktiven pharmazeutischen Inhaltsstoffs der Medikationsdosis erreicht wird und eine Rückhaltung des aktiven pharmazeutischen Inhaltsstoffs in jeder ausgewählten Trockenpulver-Inhalatorvorrichtung, in der das medizinische Produkt angewandt wird, minimiert ist.

## Revendications

1. Procédé de liaison d'une dose de médicament en poudre sèche, mesurée, comprenant au moins un ingrédient pharmaceutique actif, avec une dose d'excipient en poudre sèche, comprenant au moins un excipient biologiquement actif, dans un récipient de dosage commun, lesdites doses étant destinées à être administrées lors de l'utilisation d'un dispositif inhalateur de poudre sèche, **caractérisé par** les étapes consistant à :
choisir une formulation de la dose de médicament en poudre sèche constituée de particules de poudre inhalables, ayant un diamètre aérodynamique moyen en masse compris entre environ 0,5 µm et environ 5 µm ;
choisir une formulation dudit au moins un
excipient comprenant de grandes particules jusqu'à au moins 90 % en masse ;
définir un rapport en masse approprié entre une dose de médicament thérapeutiquement efficace choisie à remplir et la masse de la dose de l'excipient, moyennant quoi la masse de la dose d'excipient correspondante est calculée ;
mesurer séparément la dose de médicament thérapeutiquement efficace choisie et la dose calculée de l'excipient, et
remplir la dose de médicament thérapeutiquement efficace choisie, mesurée, et la dose calculée mesurée de l'excipient, éventuellement en faisant un ou plusieurs dépôts par dose, dans le récipient de dose commun.

2. Procédé selon la revendication 1, **caractérisé par** l'étape ultérieure consistant à :
mélanger les doses de médicament et d'excipient après une opération de mesure individuelle mais avant de déposer les doses mesurées dans le récipient de dosage, moyennant quoi les doses sont déjà au moins partiellement mélangées quand elles sont déposées dans le récipient.

3. Procédé selon la revendication 1, **caractérisé en ce que** ladite phase de remplissage comprend l'étape consistant à :
remplir séparément la dose de médicament thérapeutiquement efficace, choisie mesurée et la dose calculée mesurée de l'excipient, éventuellement en effectuant un ou plusieurs dépôts par dose, dans le récipient de dosage commun.

4. Procédé selon l'une quelconque des revendications 1 à 3, **caractérisé par** l'étape ultérieure consistant à :
agiter le récipient de dosage contenant les doses mesurées en utilisant l'énergie électrique ou mécanique, de sorte que les doses à l'intérieur du récipient deviennent au moins partiellement mélangées.

5. Procédé selon la revendication 1, **caractérisé par** l'étape ultérieure consistant à choisir ledit au moins un excipient dans un groupe constitué de monosaccharides, de disaccharides, de polylactides, d'oligo - et polysacch arides, de polyalcools, de polymères, de sels ou mélanges de ceux-ci.

6. Procédé selon la revendication 1 ou 5,
**caractérisé par** l'étape ultérieure consistant à choisir une formulation dudit au moins un excipient biologiquement acceptable , comprenant de grandes particules de taille supérieure à 20 µm à au moins 90 % en masse.

7. Procédé selon la revendication 1, **caractérisé par** l'étape ultérieure consistant à définir le rapport en masse entre la dose de médicament et la dose d'excipient qui doit être dans une gamme comprise entre 1:20 et 20:1.

8. Procédé selon la revendication 1, **caractérisé par** l'étape ultérieure consistant à sceller le récipient de dosage commun de façon étanche à l'humidité, en utilisant un joint extrêmement étanche.

9. Produit médical comprenant un récipient de dosage comprenant une dose de médicament en poudre sèche, comprenant au moins un ingrédient pharmaceutique actif, et comprenant en outre une dose d'excipient en poudre sèche, comprenant au moins un excipient biologiquement accept able, lesdites doses étant adaptées pour une inhalation à partir d'un récipient de dosage, en utilisant un dispositif inhalateur de poudre sèche, **caractérisé en ce que**
la dose de médicament a une masse thérapeutiquement efficace, mesurée séparément, ladite dose consistant en particules de poudre, d'un diamètre aérodynamique moyen en masse compris entre environ 0,5 µm et environ 5 µm ;
la dose d'excipient a une masse mesurée séparément calculée à partir d'un rapport de masse prédéfini relativement à la dose de médicament mesurée ; et
lesdites doses de médicament et d'excipient, éventuellement divisées en plusieurs dépôts par dose dans le récipient de dosage commun, sont agencées de façon à permettre une libération simultanée ensemble lors de l'inhalation en utilisant le dispositif inhalateur à poudre sèche.

10. Produit médical selon la revendication 9,
**caractérisé en ce que** le récipient de dosage contenant les doses mesurées est agité en utilisant l'énergie électrique ou mécanique, de sorte que les doses à l'intérieur du récipient deviennent au moins partiellement mélangées.

11. Produit médical selon la revendication 9,
**caractérisé en ce que** un rapport en masse entre la dose de médicament mesurée et la dose d'excipient est choisi dans la gamme de 1:20 - 20:1.

12. Produit médical selon la revendication 9,
**caractérisé en ce que** ledit au moins un excipient est choisi dans le groupe constitué de monosaccharides, de disaccharides, de polylactides, d'oligo- et polysaccharides, de polyalcools, de polymères, de sels ou mélanges de ceux-ci.

13. Produit médical selon la revendication 9, **caractérisé en ce que**
ledit au moins un excipient consiste en au moins 90 % en masse de particules de 20 µm de taille ou plus et éventuellement de particules ayant une taille comprise entre 0,5 et 10 µm.

14. Utilisation du produit médical selon la revendication 9, **caractérisée en ce que**
une distribution améliorée et cohérente de l'ingrédient pharmaceutique actif de la dose de médicament est atteinte et la rétention de l'ingrédient pharmaceutique actif est minimisée dans le dispositif inhalateur de poudre sèche, quel qu'il soit, où le produit médical est appliqué.
